# EUROPEAN PATENT SPECIFICATION

(11) **EP 3 842 447 B1**
(45) Date of publication and mention of the grant of the patent: **24.08.2022**
(21) Application number: 20214853.2
(22) Date of filing: 17.12.2020
(51) Int. Cl.: C07K 11/02, A61P 31/04

(54) **COMPOUND WITH ANTIBIOTIC ACTION**
VERBINDUNGEN MIT ANTIBIOTISCHER WIRKUNG
COMPOSÉ À ACTIVITÉ ANTIBIOTIQUE

(30) Priority: 23.12.2019 IT 201900025339
(43) Date of publication of application: 30.06.2021
(73) Proprietor: Naicons S.r.l., 21047 Saronno, Varese (IT)
(72) Inventor: IORIO, Marianna, 20066 Melzo, Milano (IT); SOSIO, Margherita, 20033 Solaro, Milano (IT); MAFFIOLI, Sonia Ilaria, 20127 Milano (IT); BRUNATI, Cristina, 20900 Monza (IT); DONADIO, Stefano, 21046 Malnate, Varese (IT)
(74) Representative: Long, Giorgio

(56) References cited:
- KR-A- 20110 117 922
- ANATOL LUTHER ET AL: "Advances in macrocyclic peptide-based antibiotics", BIOORGANIC & MEDICINAL CHEMISTRY, vol. 26, no. 10, 1 June 2018 (2018-06-01), pages 2850-2858, XP055724924, NL ISSN: 0968-0896, DOI: 10.1016/j.bmc.2017.08.006

## Description

### Field of the invention

The present invention relates to a compound with antibiotic action and the related production process.

### Background art

Compounds with antibiotic action, or so-called "antibiotics", are compounds capable of killing or inhibiting the growth of bacteria. The term antibiotic, in current common use, indicates a substance, of natural or synthetic origin, capable of slowing down or blocking the proliferation of bacteria. Antibiotics are therefore divided into bacteriostatic (which block the proliferation of the bacterium, preventing the cleavage thereof) and bactericidal antibiotics (which kill the microorganism). Several macrocyclic peptide-based antibiotics are known (A. Luther et al., "Advances in macrocyclic peptide-based antibiotics", Bioorganic & Medicinal Chemistry, 26(10), 2018, 2850-2858).

Antibiotics are used to treat infections caused by bacteria. However, the incorrect use thereof in time and in manner or for non-bacterial diseases reduces the effectiveness thereof, leading to the appearance of the phenomenon called "antibiotic resistance". Antibiotic resistance makes the microorganism immune to the action of the antibiotic, canceling the effects thereof.

In recent years, the phenomenon of antibiotic resistance has increased, making it necessary to continuously search for new compounds with active antibiotic action in the bacteriostatic or bactericidal action thereof.

The applicant has therefore placed the problem of identifying and characterizing a new compound which has an active antibiotic action against bacteria.

### Summary of the Invention

The object of the present invention is therefore to provide a compound with antibiotic action.

Such an object is achieved by a compound and by a process for the production of the aforesaid compound as outlined in the attached claims, the definitions of which form an integral part of the present patent application.

### Detailed description of the invention

A first object of the present invention relates to a compound of formula (I) 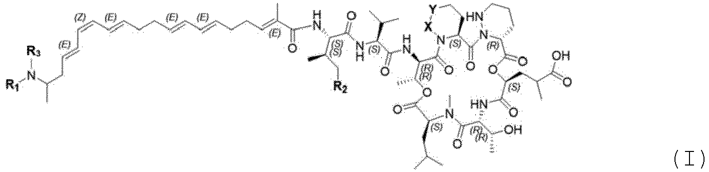 in which:
R₁ is selected from the group consisting of: H, SO₃H, C1-C6 alkyl, amino-(C1-C6)-alkyl, in which C1-C6 alkyl is preferably selected from methyl and isopropyl, and in which amino-(C1-C6)-alkyl is preferably aminoethyl;
R₂ is selected from H and C1-C3 alkyl, preferably H or CH₃;
R₃ is selected from H and C1-C3 alkyl, preferably H or CH₃;
X-Y is selected from N=CH and NH-CH₂.

Preferably the aforesaid compound is a compound in which R₁, R₂, R₃ and X-Y are as in Table 1:

**Table 1**

| **Compound** | **R₁** | **R₂** | **R₃** | **X-Y** |
|---|---|---|---|---|
| **A1** | H | H | H | N=CH |
| **A2** | H | H | H | NH-CH₂ |
| **A3** | H | CH₃ | H | N=CH |
| **A4** | H | CH₃ | H | NH-CH₂ |
| **B1** | SO₃H | H | H | N=CH |
| **B2** | SO₃H | H | H | NH-CH₂ |
| **B3** | SO₃H | CH₃ | H | N=CH |
| **B4** | SO₃H | CH₃ | H | NH-CH₂ |
| **Isopropyl**-**compound-A1** | Isopropyl | H | H | N=CH |
| **Isopropyl-compound-A2** | Isopropyl | H | H | NH-CH₂ |
| **Isopropyl-compound-A3** | Isopropyl | CH₃ | H | N=CH |
| **Isopropyl-compound-A4** | Isopropyl | CH₃ | H | NH-CH₂ |
| **Dimethyl-compound-A1** | Methyl | H | Methyl | N=CH |
| **Dimethyl-compound-A2** | Methyl | H | Methyl | NH-CH₂ |
| **Dimethyl-compound-A3** | Methyl | CH₃ | Methyl | N=CH |
| **Dimethyl-compound-A4** | Methyl | CH₃ | Methyl | NH-CH₂ |
| **Aminoethyl-compound-A1** | Aminoethyl | H | H | N=CH |
| **Aminoethyl-compound-A2** | Aminoethyl | H | H | NH-CH₂ |
| **Aminoethyl-compound-A3** | Aminoethyl | CH₃ | H | N=CH |
| **Aminoethyl-compound-A4** | Aminoethyl | CH₃ | H | NH-CH₂ |

### Table 1: some of the compounds of formula (I)

Preferably, the compound according to the present invention has a maximum wavelength (λ_{MAX}), measured by means of spectrophotometric analysis, between 230 nm and 290 nm.

The compound according to the present invention is a compound belonging to the class of acyldepsipeptide antibiotics (ADEP).

The compound is preferably characterized by a cyclic hexapeptide nucleus of alternating L- and D-amino acids, including two consecutive piperazic acids and two ester bonds. Two further exocyclic amino acids (one Val and one L-*allo*-Ile or L-Val) preferably complete the octapeptide. A C-19 olefin chain containing six double bonds (three of which are conjugated and only one in *cis* configuration) is preferably attached to the N-terminal end of the amino acid (L-allo-lle). In particular, the chain is preferably a fused (2E,6E,8E,12E,14Z,16E)-19-amino-2-methyleicosa-2,6,8,12,14,16-hexenoate as amide to the N-terminal. The amino acid sequence is preferably the following: 1) L-*allo*-isoleucine or L-valine, 2) L-valine, 3) D-threonine, 4) L-piperazic acid, 5) D-piperazic acid, 6) (2S)-2-hydroxy-4-methylglutaric acid, 7) D-threonine, 8) N-methyl-L-leucine. Hydroxy-methylglutaric acid and N-methylheucine preferably form ester bonds with the carboxyl group of piperazic acid in position 5 and with the hydroxyl group of threonine in position 3, respectively. In the ω-2 position of the chain there is preferably an amine which can be free or converted into sulfamic acid.

The compounds according to the present invention were obtained with the aid of the bacterium DSM 33314. The bacterium DSM 33314, under the Budapest Treaty, was deposited with one of the international depository authorities, recognized under the Budapest Treaty, in particular with the Leibniz-Institut DSMZ - Deutsche Sammlung von Mikroorganismen und Zellkulturen GmbH (DSMZ) in Germany.

The bacterium DSM 33314 shows a growth on solid medium typical of filamentous actinomycetes. The white-yellow colonies are characterized by a vegetative mycelium which remains tightly anchored to the solid substrate and which develops aerial mycelium and spores. The observation of these plates under an optical microscope shows spores which are arranged individually along the entire length of the aerial hyphae. It prefers a soil with an acid pH and a growth temperature of 30-37°C with good biomass production after about 6-7 days.

A second object of the present invention relates to a process for the synthesis of the compound of formula (I), comprising the steps of:
- Providing the bacterium DSM 33314;
- cultivating the bacterium DSM 33314, for a time preferably between 50 h and 72 hours, in a first culture medium comprising: glucose monohydrate, yeast extract, soy flour, C₆H₁₂NO₄SNa, NaCl;

- transferring an amount preferably between 5% and 11% of the aforesaid first culture into a second culture medium comprising: glucose monohydrate, soluble starch, yeast extract, soy flour, C₆H₁₂NO₄SNa, NaCl;
- centrifuging the aforesaid second culture so as to obtain a liquid supernatant and a solid fraction;
- carrying out, on both the supernatant and the solid fraction, a phase separation by adding a dichloromethane:methanol mixture;
- collecting and putting together the organic phases obtained from said phase separations and drying them, thus obtaining the compounds A and B of Table 1;
- adding NaBH₃CN and acetone to compounds A so as to obtain the isopropyl-compounds-A of Table 1, or adding NaBH₃CN and a 37% formaldehyde solution so as to obtain the dimethyl-compounds-A of Table 1, or adding NaBH₃CN, tert-butyl-N-(2-oxoethyl)carbamate and hydrochloric acid so as to obtain the aminoethyl-compounds-A of Table 1.

A third object of the present invention relates to a composition with antibiotic action comprising the compound according to formula (I) or of Table 1 and at least one pharmaceutically acceptable excipient.

A fourth object of the present invention relates to the use of the compound of formula (I) or of Table 1 or of the aforesaid composition with antibiotic action for use as a medicament.

A fifth object of the present invention relates to the use of the compound of formula (I) or of Table 1 or of the aforesaid composition with antibiotic action for the treatment of infections caused by bacteria.

### Brief description of the drawings

Figure 1 shows the fragmentation spectra of the compounds of formula (I).
Figure 2 shows the UV-vis spectrum of compound A3.
Figure 3 shows the 1H-NMR spectrum of the compounds A.
Figure 4 shows the HSQC spectrum of the compounds A.
Figure 5 shows the HMBC spectrum of the compounds A.
Figure 6 shows the mass spectrum of the isopropyl-compounds-A.
Figure 7 shows the mass spectrum of the dimethyl compounds-A.
Figure 8 shows the mass spectrum of the aminoethyl compounds-A.

### Embodiment examples

The following embodiment examples are provided for the sole purpose of illustration of the present invention and are not to be understood in the sense limiting the scope of protection defined by the attached claims.

### EXAMPLE 1: Method for the fermentation of the bacterium DSM 33314

The bacterium DSM 33314 was grown in a plate containing agar medium for 2-3 weeks at 28°C. The culture medium of the plate consisted of 60 g/L oatmeal, 18 g/L agar, FeSO₄ × 7 H₂O 0.001 g/L, MnCl₂ × 4 H₂O 0.001 g/L, ZnSO₄ × 7 H₂O 0.001 g/L. The microbial content (DSM 33314) of a plate was scraped and inoculated into a 50 mL flask containing 15 mL of AF/A culture medium consisting of 10 g/L glucose monohydrate, 1 g/L yeast extract, 4 g/L soy flour, 1.5 g/L MES (C₆H₁₂NO₄SNa), NaCl 0.5 g/L. The culture medium was prepared in distilled water and the pH was adjusted to 5.6 before sterilization at 121°C for 20 minutes. The inoculated flask was kept at 30°C on a rotating stirrer at 200 rpm. After 72 hours, 8% of the contents of the flask was transferred to another 500 mL flask containing 100 mL of AF/AS culture medium consisting of 10 g/L glucose monohydrate, 10 g/L soluble starch, 1 g/L yeast extract, 4 g/L soy flour, 1.5 g/L MES, 0.5 g/L NaCl. The culture medium was prepared in distilled water and the pH was adjusted to 5.6 before sterilization at 121°C for 20 minutes. The inoculated flask was kept at 30°C on a rotating stirrer at 200 rpm. The production of the compound of formula (I) was monitored by LC-MS as described below.

### EXAMPLE 2: Extraction and purification of the compound of formula (I)

The content (1 L) of 10 of the second flasks of Example 1 was centrifuged for 10 minutes at 3000 rpm in order to separate the liquid component, the so-called "clear broth" from the solid component: the bacterial component. The clear broth was placed inside a 2 L separating funnel and 300 mL of a dichloromethane:methanol (8:2) mixture was added, the separation operation was repeated three times. The organic phase obtained from the three separations was collected. 200 mL of dichloromethane:methanol mixture (8:2) was instead added to the bacterial component and the whole was kept on a tilting stirrer for 1 hour. It was subsequently centrifuged for 10 minutes at 3000 rpm. The organic liquid phase obtained from centrifugation was collected and added to the organic phase obtained from the clear broth; the two joined phases were dried under reduced pressure in a rotary evaporator. The two phases put together and dried were resuspended in 10 mL of demineralized water, sonicated for 10 minutes, transferred to glass centrifuge tubes and centrifuged for 5 minutes at 4000 rpm. The supernatant obtained from the centrifugation was removed, while the solid component underlying the supernatant was dried under vacuum, dissolved in 2 mL of dichloromethane and purified by medium-pressure chromatography using a 12 g column of normal phase silica. The column was previously conditioned with 30 mL/min of 100% phase A (dichloromethane), followed by a multi-phase gradient with increasing percentages of phase B (methanol). In particular, the chromatographic method was set with 0%, 0%, 15%, 20%, 40%, 40% methanol at, respectively, 0 minutes, 5 minutes, 15 minutes, 30 minutes, 35 minutes, 40 minutes. The fractions containing the compounds named "A" (Table 1) were put back together and concentrated under vacuum, yielding 36 mg of purified compound A (1-4). The fractions containing the compounds named "B" (Table 1) were put back together and concentrated under vacuum, yielding 8 mg of purified compound B (1-4).

The compounds A and compounds B thus obtained were analyzed by LC-MS using a C18 column maintained at 40°C with a flow rate of 0.8 mL/min. The chromatographic phases used, A and B, were 0.05% TFA in water and acetonitrile. The chromatographic analysis was conducted using a chromatograph coupled with a mass spectrometer provided with an electrospray interface (ESI) and a three-dimensional ion trap. The chromatographic analysis was carried out using a multistep program of 14 minutes set as follows: 10%, 10%, 95%, 95%, 10%, 10% phase B at, respectively, 0 minutes, 1 minute, 7 minutes, 12 minutes, 12.5 minutes, 14 minutes. The UV-VIS (190-600 nm) signals of the compounds were acquired using a diode array detector. The mass/load range (m/z) was set to 110-2000 and the ESI conditions were set as follows: spray voltage at 3500V, capillary temperature at 275°C, gas flow rate at 35 units and auxiliary gas flow rate at 15 units. Under these analytical conditions, compounds A1 and A2 showed a retention time (Rt) of 6.6 minutes, compounds A3 and A4 showed an Rt of 6.8 minutes, compounds B1 and B2 showed an Rt of 7.8 minutes and compounds B3 and B4 showed an Rt of 8.0 minutes.

### EXAMPLE 3: Physical-chemical characterization of the compounds of formula (I)

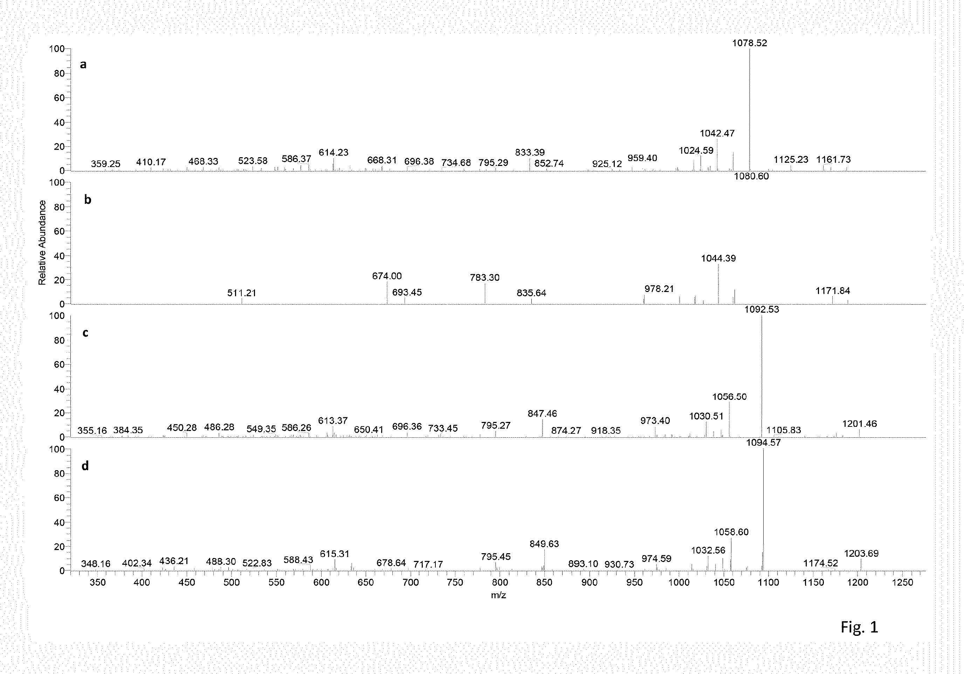

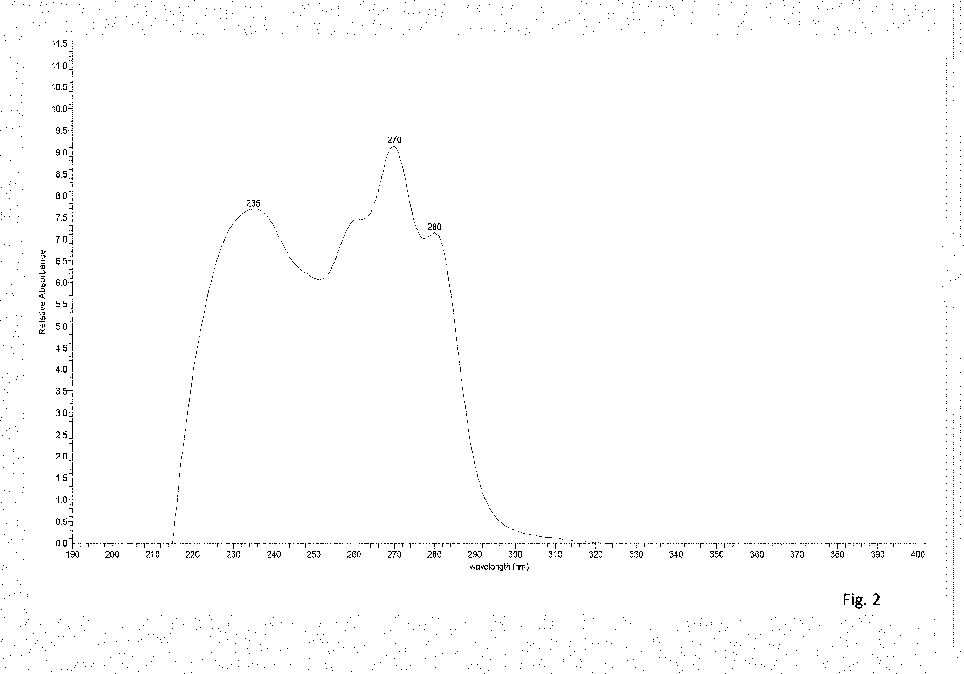

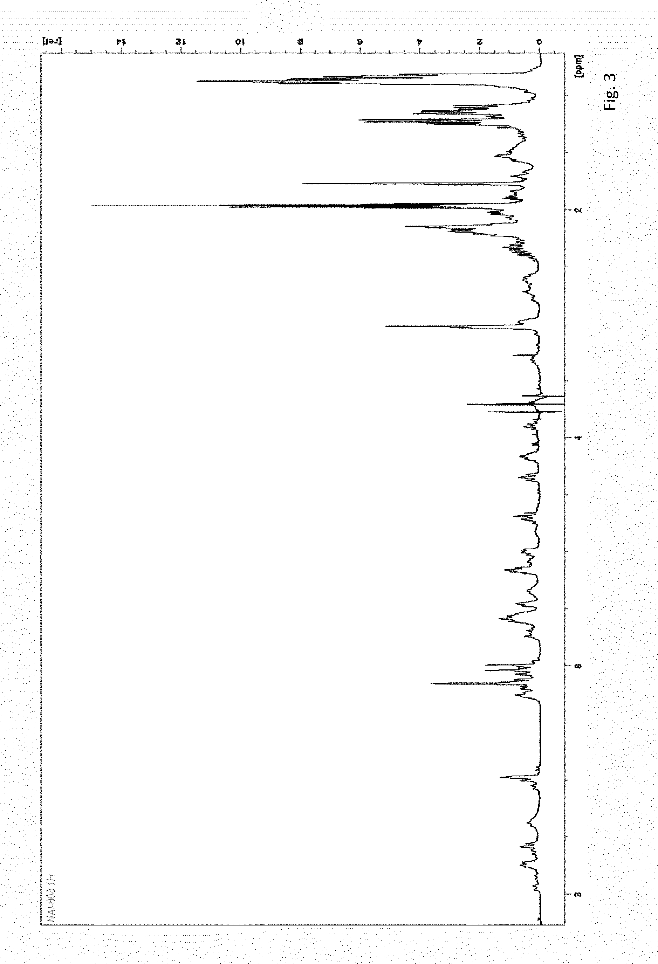

A) Mass spectrometry:
   in the mass spectrometry analysis compound A1 provided a doubly protonated ion having an m/z ratio = 603 [M + 2H]²⁺, a monoprotonated ion having an m/z ratio = 1205 [M + H]⁺ and the following main fragments: m/z 1078, 833, 795 and 607. Compound A2 provided a doubly protonated ion having m/z ratio = 604 [M + 2H]²⁺, a monoprotonated ion having m/z ratio = 1207 [M + H]⁺ and the following main fragments: m/z 1080, 835, 797 and 615. Compound A3 provided a doubly protonated ion having m/z ratio = 610 [M + 2H]²⁺, a monoprotonated ion having m/z ratio = 1219 [M + H]⁺ and the following main fragments: m/z 1092, 847, 795 and 613. Compound A4 provided a doubly protonated ion having m/z ratio = 611 [M + 2H]²⁺, a monoprotonated ion having m/z ratio = 1221 [M + H]⁺ and the following main fragments: m/z 1094, 849, 797 and 615. The fragmentation spectra are shown in Figure 1, in which the relative intensities are shown on the ordinate axes, while on the abscissa axes the m/z ratios are shown and in which, respectively: a) corresponds to the spectrum of compound A1, b) corresponds to the spectrum of compound A2, c) corresponds to the spectrum of compound A3 and d) corresponds to the spectrum of compound A4. Under the same conditions: the compounds B1, B2, B3 and B4 provided monoprotonated ions having m/z ratio = 1285, 1287, 1299 and 1301, respectively, but none of them provided fragments when subjected to a maximum collision energy of 30 eV.
B) UV-vis spectroscopy:
   the UV-vis spectra of the compounds of formula (I) are comparable to each other. The analysis of the UV-vis spectra was performed in a solution of 0.05% trifluoroacetic acid (TFA) in water/acetonitrile (in a 40:60 ratio), with a diode array detector. The UV-vis spectra showed an absorbance peak at 235 nm followed by three further peaks at 260 nm, 270 nm and 280 nm. Figure 2 shows the UV-vis spectrum of compound A3. In the above Figure, the abscissa axis represents the wavelength (nm), while the ordinate axis represents the relative absorbance.
C) NMR spectroscopy:
   The NMR spectroscopic analysis was carried out by 1H-NMR analysis, 13C-NMR analysis and 2D NMR analysis. The NMR spectra were recorded using the mixtures CD₃CN/D ₂O 8/2 (v/v) at 25°C. The residual acetonitrile signal at 1.99 ppm was used as an internal standard. Figure 3 shows the 1H-NMR spectrum of the compounds A. The 1H NMR spectrum of Figure 3 shows the following signal groups (in ppm), at 300 MHz, using CD₃CN as internal standard (1.99 ppm), [δ = ppm, multiplicity; (Attribution)] :
   0.81 - 1.76 (CH₃), 1.10 - 2.99 (beta, gamma and delta CH₂), 1.22 - 3.29 (aliphatic CH, CH₂ and CH₃ of the chain), 4.15 - 5.45 (CH alpha), 1.44 - 3.88 (CH beta and gamma), 5.56 - 6.24 (olefin CH of the chain), 6.95 (CH delta of unsaturated piperazic acid in position 4).

The compounds A also showed the following groups of 13C signals [δ = ppm; (attribution)]: 11.0 - 30.7 (CH₃), 18.1 - 46.2 (beta, gamma and delta CH₂), 17.3 - 47.8 (aliphatic CH, CH₂ and CH₃ of the chain), 50.3 - 73.2 (alpha CH), 24.2 - 67.4 (beta and gamma CH), 126.8 - 136.2 (olefin CH of the chain), 145.6 (delta CH of unsaturated piperazic acid in position 4), 169 - 178 (peptide carbonyls and carboxylic acid).

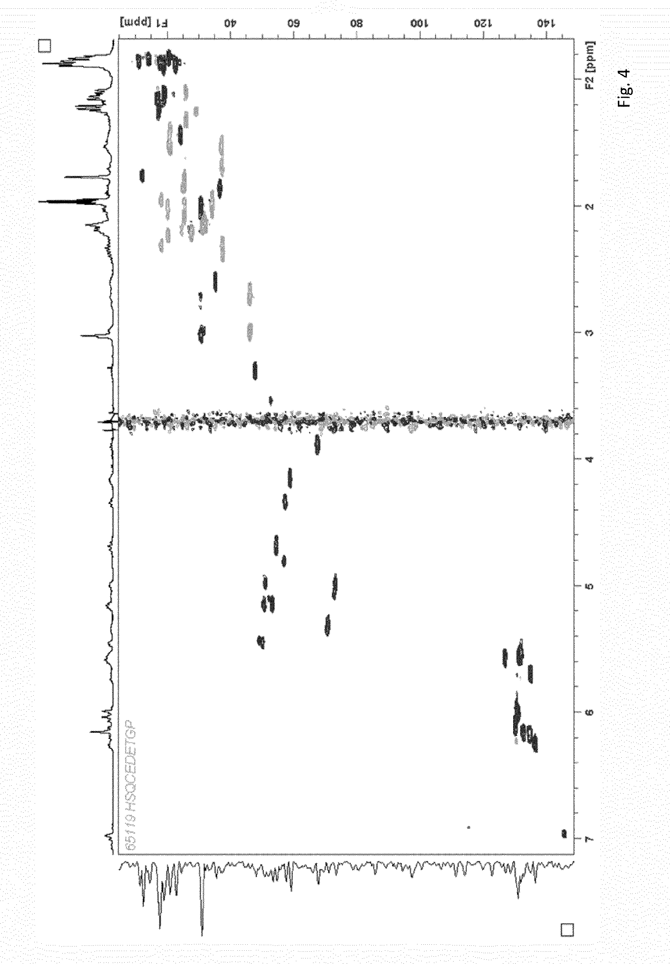

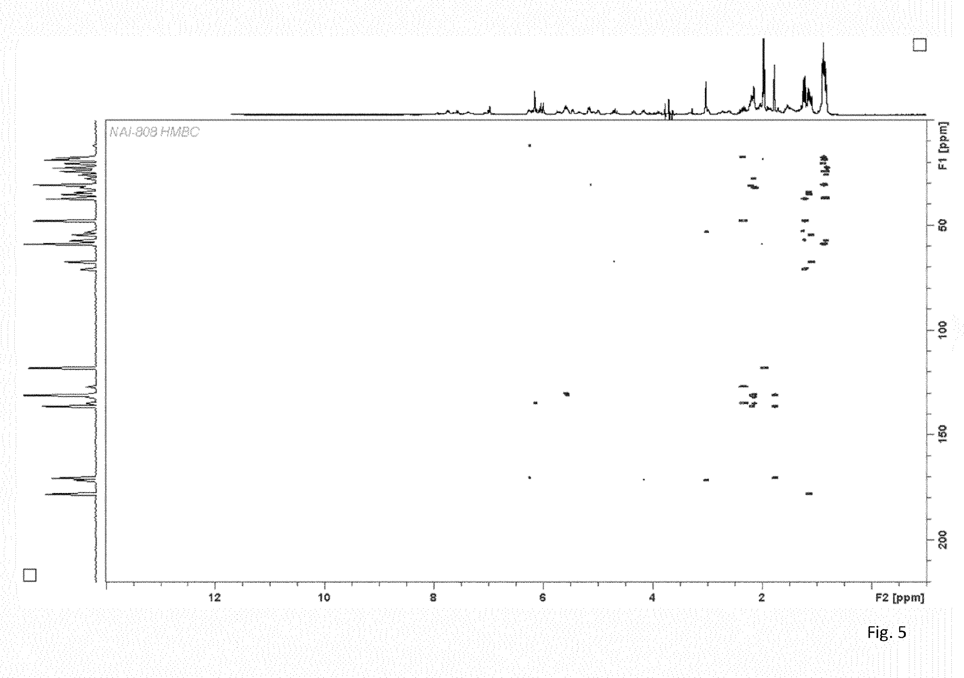

Figures 4 and 5 show, respectively, the HSQC and HMBC spectra of the compounds A.

### EXAMPLE 4: Synthesis of isopropyl-compounds-A

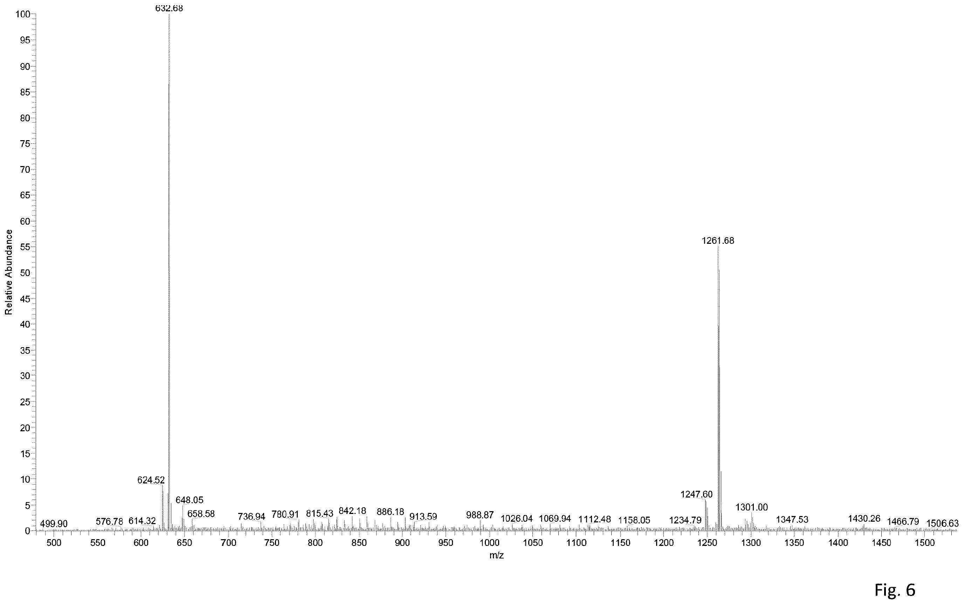

To a solution of 1 mg of the compounds A (20% A1-2 and 80% A3-4) in 50 µL of methanol and 450 µL of acetone, 1 mg of NaBH₃CN was added and the reaction mixture was kept under stirring at room temperature for 30 minutes. The reaction was extinguished by evaporating the solvents under reduced pressure. The compounds obtained, i.e., the isopropyl-compounds-A, showed peaks of monoprotonated ions having m/z ratio 1247, 1249, 1261 and 1263 [M + H]⁺ in a ratio 1:1:4:4 and doubly protonated ion peaks having m/z ratio 624, 625, 632 and 633 [M + 2H]²⁺. Figure 6 shows the mass spectrum of the isopropyl-compounds-A, in which the m/z ratio is shown on the abscissa axis, while the relative intensity is shown on the ordinate axis.

### EXAMPLE 5: Synthesis of dimethyl-compounds-A

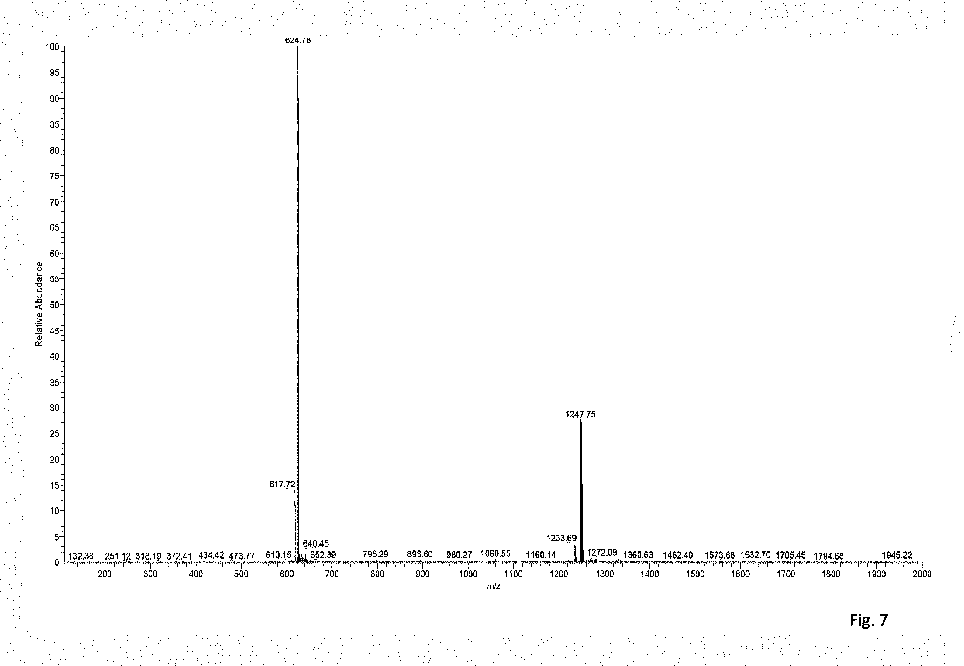

To a solution of 1 mg of the compounds A (20% A1-2 and 80% A3-4) in 500 µL of methanol, 5 µL of 37% formaldehyde solution and 1 mg of NaBH₃CN were added. The reaction mixture was kept under stirring at room temperature for 30 minutes. The reaction was stopped by evaporating the solvents under reduced pressure. The compounds obtained, i.e., the dimethyl-compounds-A, showed peaks of monoprotonated ions having m/z ratio 1233, 1235, 1247 and 1249 [M + H]⁺ in a 1:1:4:4 ratio and doubly protonated ion peaks having m/z ratio 617, 618, 624 and 625 [M + 2H]²⁺. Figure 7 shows the mass spectrum of the dimethyl-compounds-A.

### EXAMPLE 6: Synthesis of aminoethyl-compounds-A

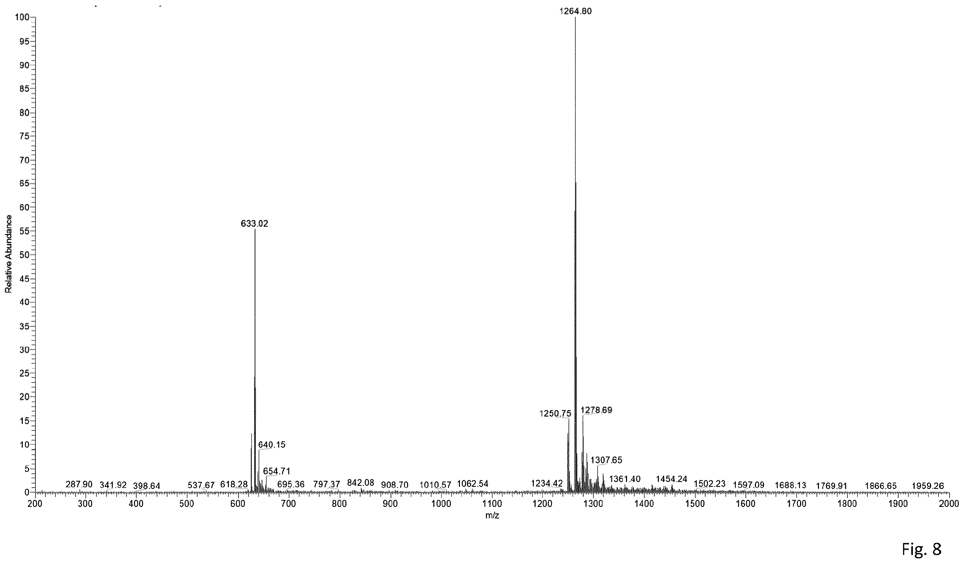

To a solution of 1 mg of the compounds A (20% A1-2 and 80% A3-4) in 500 µL of methanol, 1 mg of tert-butyl-N-(2-oxoethyl) carbamate and 1 mg of NaBH₃CN were added and the reaction mixture was kept under stirring at room temperature for 16 hours. The solution was evaporated to dryness under reduced pressure. A solution of 500 µL of acetonitrile and 100 µL of 6N hydrochloric acid was added to de-protect the derivative. After 5 minutes a solution of 500 µL of water and 100 µL of 6N sodium hydroxide was added to stop the reaction and neutralize. The compounds obtained, i.e., the aminoethyl-compounds-A, showed peaks of monoprotonated ions having m/z ratio 1249, 1251, 1263 and 1265 [M + H]⁺ in a 1:1:4:4 ratio and doubly protonated ion peaks having m/z ratio 625, 626, 632 and 633 [M + 2H]²⁺. Figure 8 shows the mass spectrum of the aminoethyl-compounds-A.

### EXAMPLE 7: Antibiotic action, in vitro, of the compounds of formula (I)

Minimum inhibitory concentrations (MIC), for aerobic and anaerobic bacteria, were determined by the method of micro-dilution of the culture broth, according to the guidelines of the Clinical and Laboratory Standards Institute (CLSI M100-S16 and M27-A, NCCLS, Wayne, PA), using 1×10⁵ CFU/mL as inoculum for all the microorganisms, except for *Candida albicans* which was inoculated 1×10⁴ CFU/mL.

Gram positive and Gram negative bacteria were cultured in Mueller - Hinton medium with cation supplement (the Mueller - Hinton medium was prepared by adding CaCl₂ and MgCl₂ at final concentrations of 20 mg/mL and 10 mg/mL, respectively). Only *Streptococcus pyogenes* and *Streptococcus pneumoniae* were cultivated in Todd Hewitt culture medium.

*Mycobacterium smegmatis* and *Candida albicans* were cultivated in Middlebrook 7H9 culture medium with the addition of OADC growth supplement and RPMI 1640 medium.

The MICs for the anaerobic bacteria were determined by diluting the culture broth with Brucella medium (BB) added with hemin (5 µg/mL), vitamin K1 (1 µg/mL), lysed horse blood (5%) and oxidase (1:25 v/v).

The test results were evaluated after 24 hours of incubation at 37°C for all strains tested except for *Mycobacterium smegmatis, Clostridium difficile* and *Candida albicans,* which required 48 hours of culture.

The compounds of formula (I) and the known substances, gentamicin (GEN) and vancomycin (VA), used as control, were dissolved in 100% dimethyl sulfoxide in order to obtain a stock solution of 10 mg/mL and, subsequently, were diluted in the test medium, defined above, to obtain the working solutions.

The test results are shown in Tables 2 and 3.

### Table 2

**Table 2: antibiotic action of compounds A and B**

| **Strain** | **MIC (µg/mL)** | | | |
|---|---|---|---|---|
| | **Compounds A** | **Compounds B** | **GEN** | **VA** |
| *Staphylococcus aureus* Met-S | ≤0.06 | >128 | 0.25 | 0.25 |
| *S. aureus Met-R* | ≤0.06 | >128 | >128 | 0.5 |
| *S. aureus* Glycopeptide- Intermediate | >128 | >128 | 64 | 8 |
| *S. epidermidis* | ≤0.06 | >128 | ≤0.06 | 1 |
| *Streptococcus pneumoniae Felton* UC41 | ≤0.06 | >128 | 2 | 0.25 |
| *S. pyogenes* C203 | ≤0.06 | >128 | 2 | 0.25 |
| *Micrococcus luteus* | 1 | >128 | 0.25 | 0.125 |
| *Bacillus subtilis* 168 | 0.125 | >128 | ≤0.06 | 0.25 |
| *Enterococcus faecalis* Van-S | 8 | >128 | 4 | 1 |
| *Enterococcus faecalis* Van-A | 32 | >128 | 4 | >128 |
| *Enterococcus faecium* Van-S | 8 | >128 | 16 | 4 |
| *Enterococcus faecium* Van-A | 2 | >128 | 64 | >128 |
| *Moraxella catarrhalis* U503 | >128 | >128 | 0.125 | >128 |
| *Pseudomonas aeruginosa* | >128 | >128 | 1 | >128 |
| *Escherichia coli* MG1655 *hypersensitive* | >128 | >128 | 0.5 | >128 |
| *Escherichia coli* | >128 | >128 | 1 | >128 |
| *Candida albicans* SKF2270 | >128 | >128 | >128 | >128 |
| *Clostridium difficile* 421 | ≤0.06 | >128 | 64 | 1 |
| *Mycobacterium smegmatis* mc² 155 | >128 | >128 | 2 | 32 |

### Table 3

**Table 3: antibiotic action of some compounds of formula (I)**

| **Strain** | **MIC (µg/mL)** | | | |
|---|---|---|---|---|
| | **Compounds A** | **Isopropyl-compounds-A** | **Dimethyl-compounds-A** | **Aminoethyl compounds-A** |
| *Staphylococcus aureus* Met-S | ≤0.06 | 0.25 | 0.5 | 0.78 |
| *S. aureus Met-R* | ≤0.06 | 4 | 8 | 0.1 |
| *S. aureus* Glycopeptide-Intermediate | >128 | 4 | 4 | 25 |
| *S. epidermidis* | ≤0.06 | 2 | 2 | 0.78 |
| *Streptococcus pneumoniae Felton* UC41 | 0.25 | 4 | 32 | 0.05 |
| *S. pyogenes* C203 | ≤0.06 | 1 | 8 | 8 |
| *Bacillus subtilis* 168 | 0.25 | 1 | 4 | nd |
| *Enterococcus faecium* Van-S | 1 | 16 | >128 | 25 |
| *Enterococcus faecium* Van-A | 0.5 | 4 | >128 | 12.5 |
| *Moraxella catarrhalis* U503 | >128 | >128 | >128 | 0.05 |
| *Pseudomonas aeruginosa* | >128 | >128 | >128 | nd |
| *Escherichia coli* MG1655 *hypersensitive* | >128 | >128 | >128 | >128 |
| *Candida albicans* SKF2270 | >128 | >128 | >128 | nd |

From these analyses it is clear that the compound of formula (I) has an active antibiotic action against bacteria, thus proving to be a new compound suitable for counteracting the antibiotic resistance phenomenon.

## Claims

1. A compound of formula (I) 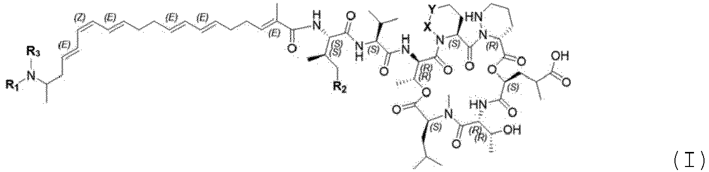 wherein:
R₁ is selected from the group consisting of: H, SO₃H, C1-C6 alkyl, amino-(C1-C6)-alkyl, wherein C1-C6 alkyl is preferably selected from methyl and isopropyl, and wherein amino-(C1-C6)-alkyl is preferably aminoethyl;
R₂ is selected from H and C1-C3 alkyl, preferably H or CH₃;
R₃ is selected from H and C1-C3 alkyl, preferably H or CH₃;
X-Y is selected from N=CH and NH-CH₂.

2. The compound according to claim 1, wherein R₁, R₂, R₃ and X-Y are as in Table 1:
**Table 1**
| **Compound** | **R₁** | **R₂** | **R₃** | **X-Y** |
|---|---|---|---|---|
| **A1** | H | H | H | N=CH |
| **A2** | H | H | H | NH-CH₂ |
| **A3** | H | CH₃ | H | N=CH |
| **A4** | H | CH₃ | H | NH-CH₂ |
| **B1** | SO₃H | H | H | N=CH |
| **B2** | SO₃H | H | H | NH-CH₂ |
| **B3** | SO₃H | CH₃ | H | N=CH |
| **B4** | SO₃H | CH₃ | H | NH-CH₂ |
| **Isopropyl-compound-A1** | Isopropyl | H | H | N=CH |
| **Isopropyl-compound-A2** | Isopropyl | H | H | NH-CH₂ |
| **Isopropyl-compound-A3** | Isopropyl | CH₃ | H | N=CH |
| **Isopropyl-compound-A4** | Isopropyl | CH₃ | H | NH-CH₂ |
| **Dimethyl-compound-A1** | Methyl | H | Methyl | N=CH |
| **Dimethyl-compound-A2** | Methyl | H | Methyl | NH-CH₂ |
| **Dimethyl-compound-A3** | Methyl | CH₃ | Methyl | N=CH |
| **Dimethyl-compound-A4** | Methyl | CH₃ | Methyl | NH-CH₂ |
| **Aminoethyl-compound-A1** | Aminoethyl | H | H | N=CH |
| **Aminoethyl-compound-A2** | Aminoethyl | H | H | NH-CH₂ |
| **Aminoethyl-compound-A3** | Aminoethyl | CH₃ | H | N=CH |
| **Aminoethyl-compound-A4** | Aminoethyl | CH₃ | H | NH-CH₂ |

3. The compound according to claim 1 or 2, wherein the compound has a maximum wavelength (λ_{MAX}), measured by means of spectrophotometric analysis, comprised between 230 nm and 290 nm.

4. A process for the synthesis of the compound according to claim 1 or 2, comprising the steps of:
- providing the bacterium DSM 33314;
- cultivating the bacterium DSM 33314, for a time comprised between 50 h and 72 h, in a first culture medium comprising: glucose monohydrate, yeast extract, soy flour, C₆H₁₂NO₄SNa, NaCl;
- transferring an amount comprised between 5% and 11% of said first culture into a second culture medium comprising: glucose monohydrate, soluble starch, yeast extract, soy flour, C₆H₁₂NO₄SNa, NaCl;
- centrifuging said second culture so as to obtain a liquid supernatant and a solid fraction;
- carrying out, on both the supernatant and the solid fraction, a phase separation by adding a dichloromethane:methanol mixture;
- collecting and putting together the organic phases obtained from said phase separations and drying them, thus obtaining the compounds A and B according to claim 2;
- adding NaBH₃CN and acetone to compounds A so as to obtain the isopropyl-compounds-A according to claim 2, or adding NaBH₃CN and a 37% formaldehyde solution so as to obtain the dimethyl-compounds-A according to claim 2, or adding NaBH₃CN, tert-butyl-N-(2-oxoethyl)carbamate and hydrochloric acid so as to obtain the aminoethyl-compounds-A according to claim 2.

5. A composition with antibiotic action comprising the compound according to claim 1 or 2 and at least one pharmaceutically acceptable excipient.

6. A compound according to claim 1 or 2 or a composition according to claim 5 for use as a medicament.

7. The compound according to claim 6 or the composition according to claim 6 for use in the treatment of infections caused by bacteria.

## Patentansprüche

1. Verbindung gemäß Formel (I) 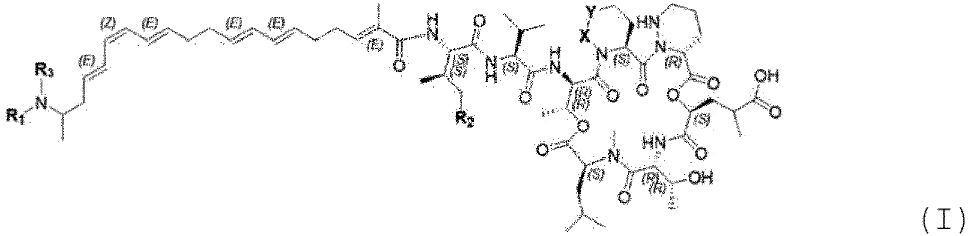 wobei:
R₁ ausgewählt ist aus der Gruppe bestehend aus: H, SO₃H, C₁-C₆ Alkyl, Amino-(C₁-C₆)-Alkyl, wobei C₁-C₆ Alkyl vorzugsweise ausgewählt ist aus Methyl und Isopropyl, und wobei Amino-(C₁-C₆)-Alkyl vorzugsweise Aminoethyl ist;
R₂ ausgewählt ist aus H und C₁-C₃ Alkyl, vorzugsweise H oder CH₃;
R₃ ausgewählt ist aus H und C₁-C₃ Alkyl, vorzugsweise H oder CH₃;
X-Y ausgewählt ist aus N=CH und NH-CH₂.

2. Verbindung nach Anspruch 1, wobei R₁, R₂, R₃ und X-Y wie in Tabelle 1 sind:
**Tabelle 1**
| **Verbindung** | **R₁** | **R₂** | **R₃** | **X-Y** |
|---|---|---|---|---|
| **A1** | H | H | H | N=CH |
| **A2** | H | H | H | NH-CH₂ |
| **A3** | H | CH₃ | H | N=CH |
| **A4** | H | CH₃ | H | NH-CH₂ |
| **B1** | SO₃H | H | H | N=CH |
| **B2** | SO₃H | H | H | NH-CH₂ |
| **B3** | SO₃H | CH₃ | H | N=CH |
| **B4** | SO₃H | CH₃ | H | NH-CH₂ |
| **Isopropyl-Verbindung-A1** | Isopropyl | H | H | N=CH |
| **Isopropyl-Verbindung-A2** | Isopropyl | H | H | NH-CH₂ |
| **Isopropyl-Verbindung-A3** | Isopropyl | CH₃ | H | N=CH |
| **Isopropyl-Verbindung-A4** | Isopropyl | CH₃ | H | NH-CH₂ |
| **Dimethyl-Verbindung-A1** | Methyl | H | Methyl | N=CH |
| **Dimethyl-Verbindung-A2** | Methyl | H | Methyl | NH-CH₂ |
| **Dimethyl-Verbindung-A3** | Methyl | CH₃ | Methyl | N=CH |
| **Dimethyl-Verbindung-A4** | Methyl | CH₃ | Methyl | NH-CH₂ |
| **Aminoethyl-Verbindung-A1** | Aminoethyl | H | H | N=CH |
| **Aminoethyl-Verbindung-A2** | Aminoethyl | H | H | NH-CH₂ |
| **Aminoethyl-Verbindung-A3** | Aminoethyl | CH₃ | H | N=CH |
| **Aminoethyl-Verbindung-A4** | Aminoethyl | CH₃ | H | NH-CH₂ |

3. Verbindung nach Anspruch 1 oder 2, wobei die Verbindung eine maximale Wellenlänge (λ_{MAX}) aufweist, gemessen mittels Spektrophotometrieanalyse, die zwischen 230 nm und 290 nm umfasst ist.

4. Verfahren für die Synthese der Verbindung nach Anspruch 1 oder 2, umfassend die Schritte:
- Bereitstellen des Bakteriums DSM 33314;
- Kultivieren des Bakteriums DSM 33314, für eine Dauer umfassend zwischen 50 h und 72 h, in einem ersten Medium umfassend: Glukosemonohydrat, Hefeextrakt, Sojamehl, C₆H₁₂NO₄SNa, NaCl;
- Transferieren einer Menge umfassend zwischen 5 % und 11 % der ersten Kultur in ein zweites Kulturmedium umfassend: Glukosemonohydrat, lösliche Stärke, Hefeextrakt, Sojamehl, C₆H₁₂NO₄SNa, NaCl;
- Zentrifugieren der zweiten Kultur, um einen flüssigen Überstand und einen festen Teil zu erhalten;
- Ausführen einer Phasentrennung an sowohl dem Überstand als auch dem festen Teil, durch Zugeben eines Dichlormethan:Methanol-Gemischs;
- Sammeln und Zusammenbringen der organischen Phasen, die durch die Phasentrennungen erhalten wurden und Trocknen derselben, und dadurch Erhalten der Verbindungen A und B nach Anspruch 2;
- Zugeben von NaBH₃CN und Aceton zu Verbindungen A, um so die Isopropyl-Verbindungen-A nach Anspruch 2 zu erhalten, oder Zugeben von NaBH₃CN und eine 37 %-ige Formaldehydlösung, um so die Dimethyl-Verbindungen-A nach Anspruch 2 zu erhalten, oder Zugeben von NaBH₃CN, tert-Butyl-N-(2-oxoethyl)carbamat und Salzsäure, um so die Aminoethyl-Verbindungen-A nach Anspruch 2 zu erhalten.

5. Zusammensetzung mit antibiotischer Wirkung, die die Verbindung nach Anspruch 1 oder 2 und mindestens einen pharmazeutisch annehmbaren Hilfsstoff umfasst.

6. Verbindung nach Anspruch 1 oder 2 oder eine Zusammensetzung nach Anspruch 5 zur Verwendung als ein Medikament.

7. Verbindung nach Anspruch 6 oder die Zusammensetzung nach Anspruch 6 zur Verwendung bei der Behandlung von durch Bakterien verursachten Infektionen.

## Revendications

1. Composé de formule (I) 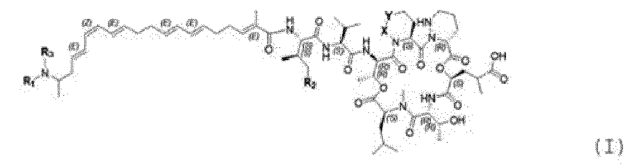 dans lequel :
R₁ est choisi dans le groupe constitué de : H, SO₃H, alkyle en C1-C6, amino-alkyle en (C1-C6), dans lequel l'alkyle en C1-C6 est de préférence choisi parmi un méthyle et un isopropyle, et dans lequel l'amino-alkyle en (C1-C6) est de préférence un aminoéthyle ;
R₂ est choisi parmi un H et un alkyle en C1-C3, de préférence un H ou un CH₃;
R₃ est choisi parmi un H et un alkyle en C1-C3, de préférence un H ou un CH₃ ;
X-Y est choisi parmi un N=CH et un NH-CH₂.

2. Composé selon la revendication 1, dans lequel R₁, R₂, R₃ et X-Y sont tels que dans le Tableau 1 :
**Tableau 1**
| **Composé** | **R₁** | **R₂** | **R₃** | **X-Y** |
|---|---|---|---|---|
| **A1** | H | H | H | N=CH |
| **A2** | H | H | H | NH-CH₂ |
| **A3** | H | CH₃ | H | N=CH |
| **A4** | H | CH₃ | H | NH-CH₂ |
| **B1** | SO₃H | H | H | N=CH |
| **B2** | SO₃H | H | H | NH-CH₂ |
| **B3** | SO₃H | CH₃ | H | N=CH |
| **B4** | SO₃H | CH₃ | H | NH-CH₂ |
| **Isopropyl-composé-A1** | Isopropyl | H | H | N=CH |
| **Isopropyl-composé-A2** | Isopropyl | H | H | NH-CH₂ |
| **Isopropyl-composé-A3** | Isopropyl | CH₃ | H | N=CH |
| **Isopropyl-composé-A4** | Isopropyl | CH₃ | H | NH-CH₂ |
| **Diméthyl-composé-A1** | Méthyl | H | Méthyl | N=CH |
| **Diméthyl-composé-A2** | Méthyl | H | Méthyl | NH-CH₂ |
| **Diméthyl-composé-A3** | Méthyl | CH₃ | Méthyl | N=CH |
| **Diméthyl-composé-A4** | Méthyl | CH₃ | Méthyl | NH-CH₂ |
| **Aminoéthyl-composé-A1** | Aminoéthyl | H | H | N=CH |
| **Aminoéthyl-composé-A2** | Aminoéthyl | H | H | NH-CH₂ |
| **Aminoéthyl-composé-A3** | Aminoéthyl | CH₃ | H | N=CH |
| **Aminoéthyl-composé-A4** | Aminoéthyl | CH₃ | H | NH-CH₂ |

3. Composé selon la revendication 1 ou 2, dans lequel le composé a une longueur d'onde maximale (λ_{MAX}), mesurée au moyen d'une analyse spectrophotométrique, comprise entre 230 nm et 290 nm.

4. Procédé pour la synthèse du composé selon la revendication 1 ou 2, comprenant les étapes de :
- fourniture de la bactérie DSM 33314;
- culture de la bactérie DSM 33314, pendant un temps compris entre 50 h et 72 h, dans un premier milieu de culture comprenant : du glucose monohydraté, un extrait de levure, de la farine de soja, du C₆H₁₂NO₄SNa, du NaCl;
- transfert d'une quantité comprise entre 5 % et 11 % de ladite première culture dans un second milieu de culture comprenant : du glucose monohydraté, de l'amidon soluble, un extrait de levure, de la farine de soja, du C₆H₁₂NO₄SNa, du NaCl;
- centrifugation de ladite seconde culture de façon à obtenir un surnageant liquide et une fraction solide ;
- réalisation, à la fois sur le surnageant et sur la fraction solide, d'une séparation de phase par ajout d'un mélange dichlorométhane:méthanol ;
- collecte et regroupement des phases organiques obtenues à partir desdites séparations de phase et leur séchage, pour ainsi obtenir les composés A et B selon la revendication 2 ;
- ajout de NaBH₃CN et d'acétone aux composés A de façon à obtenir les isopropyl-composés-A selon la revendication 2, ou ajout de NaBH₃CN et d'une solution de formaldéhyde à 37 % de façon à obtenir les diméthyl-composés-A selon la revendication 2, ou ajout de NaBH₃CN, de tert-butyl-N-(2-oxoéthyl)carbamate et d'acide chlorhydrique de façon à obtenir les aminoéthyl-composés-A selon la revendication 2.

5. Composition à action antibiotique comprenant le composé selon la revendication 1 ou 2 et au moins un excipient pharmaceutiquement acceptable.

6. Composé selon la revendication 1 ou 2 ou une composition selon la revendication 5 pour une utilisation en tant que médicament.

7. Composé selon la revendication 6 ou la composition selon la revendication 6 pour une utilisation dans le traitement d'infections causées par des bactéries.


### SEQUENCE LISTING

<110> Naicons S.r.l.
<120> COMPOSTO AD AZIONE ANTIBIOTICA
<130> @I0184714/RAS
<160> 1
<170> PatentIn version 3.5
<210> 1
   <211> 8
   <212> PRT
   <213> Artificial Sequence
<220>
   <223> -
<220>
   <221> MOD_RES
   <222> (1) .. (1)
   <223> L-allo-isoleucina oppure L-valina
<220>
   <221> MOD_RES
   <222> (4) .. (4)
   <223> acido L-piperazico
<220>
   <221> MOD_RES
   <222> (5) .. (5)
   <223> acido D-piperazico
<220>
   <221> MOD_RES
   <222> (6) .. (6)
   <223> acido (2S)-2-idrossi-4-metilglutarico
<220>
   <221> MOD_RES
   <222> (8) .. (8)
   <223> N-metil-L-leucina
<400> 1
   Xaa Val Thr Xaa Xaa Xaa Thr Leu
   1               5